# EUROPÄISCHE PATENTSCHRIFT

(11) **EP 1 749 100 B1**
(45) Veröffentlichungstag und Bekanntmachung des Hinweises auf die Patenterteilung: **12.11.2008**
(21) Anmeldenummer: 05715924.6
(22) Anmeldetag: 10.03.2005
(51) Int. Cl.: C12P 9/00

(54) **VERFAHREN ZUM ANAEROBEN BIOLOGISCHEN ABBAU VON ORGANOSILOXANEN**
METHOD FOR EFFECTING THE ANAEROBIC BIOLOGICAL DECOMPOSITION OF ORGANOSILOXANES
PROCEDE DE BIODEGRADATION ANAEROBIE D'ORGANOSILOXANES

(30) Priorität: 11.03.2004 DE 102004011993
(43) Veröffentlichungstag der Anmeldung: 07.02.2007
(73) Patentinhaber: Wacker Chemie AG, 81737 München (DE)
(72) Erfinder: CANDUSSIO, Anton, 81825 München (DE); AMANN, Manfred, 85235 Odelzhausen (DE); WICH, Günter, 81377 München (DE); HEIDER, Johann, 79100 Freiburg (DE)
(74) Vertreter: Potten, Holger
(86) Internationale Anmeldenummer: PCT/EP2005/002546
(87) Internationale Veröffentlichungsnummer: WO 2005/087939

(56) Entgegenhaltungen:
- US-A- 6 020 184
- GRÜMPING, R. ET AL.: "Microbial Degradation of Octamethylcyclotetrasiloxane" APPLIED AND ENVIRONMENTAL MICROBIOLOGY, Bd. 65, Nr. 5, Mai 1999 (1999-05), Seiten 2276-2278, XP002333081
- SABOURIN, C.L. ET AL.: "Biodegradation of Dimethylsilanediol in Soils" APPLIED AND ENVIRONMENTAL MICROBIOLOGY, Bd. 62, Nr. 12, Dezember 1996 (1996-12), Seiten 4352-4360, XP002944438
- LUKASIAK, J. ET AL.: "Biodegradation of silicones (organosiloxanes)" BIOPOLYMERS, Bd. 9, 2003, Seiten 539-568, XP008048836
- WATTS, R.J. ET AL.: "Fate and effects of polydimethylsiloxanes on pilot and bench-top activated sludge reactors and anaerobic/aerobic digesters" WATER RESEARCH, Bd. 29, Nr. 10, Oktober 1995 (1995-10), Seiten 2405-2411, XP004035183
- HOLLIGER, C. ET AL.: "Contaminated environments in the subsurface and bioremediation: organic compounds" FEMS MICROBIOLOGY REVIEWS, Bd. 20, Nr. 3-4, Juli 1997 (1997-07), Seiten 517-523, XP002333082

## Beschreibung

Die Erfindung betrifft den anaerober Abbau von linearen oder cyclischen Polyorganosiloxanen, wie z.B. Polydimethylsiloxan (PDMS) oder organofunktionellen Siloxanen, von Organosilanen insbesondere Organosilanolen und von über chemische Depolymerisation aus diesen Verbindungen gebildeten Bruchstücken.

Jährlich werden mehrere 100.000 Tonnen von Polymeren auf Basis von Polydimethylsiloxan (PDMS), basierend auf einer - (Si-O-Si) - Wiederholungseinheit, hergestellt. Ein großer Teil dieser Siloxane gelangt bei oder nach der Anwendung (Textilindustrie, Waschmittel, Papierindustrie, Kosmetik, Bau, Pharma, Agro, Petro etc.) in die Umwelt. Bei Siloxanen handelt es sich um Polymere, die natürlicherweise nicht vorkommen. Bisher sind auch keine biologischen Prozesse bekannt, die eine Si-C - Verbindung zwischen einem Silizium - Atom und dem Kohlenstoffatom einer Methylgruppe bilden oder spalten. Verfahren zum biologischen Siloxanabbau in Abwässern z.B in kommunalen Kläranlagen oder in Abwasserbehandlungseinrichtungen der chemischen Industrie, in Böden, Sedimenten, Schlämmen oder anderen Umweltkompartimenten sind nicht bekannt.

Gravier et al. (2003) beschreiben zusammenfassend, wie Siloxanpolymeren in der Umwelt chemisch abgebaut werden. Es kommt nicht zu einer Anreicherung der hochmolekularen Siloxane, sondern diese werden im wesentlichen durch Hydrolyse in wässrigen oder terrestrischen Habitaten zu Organosilanol-terminierten Oligomeren abgebaut. Diese Organosilanole und niedermolekulare PDMS-Bruchstücke so wie cyclische Siloxane verdampfen in die Atmosphäre, wo sie letztlich durch die dort vorhandenen Hydroxylradikale zu Silikat, CO₂ und Wasser oxidiert werden.

Hochmolekulares Polyorganosiloxan ist nicht wasserlöslich. In wässrigen Systemen oder im Abwasser kommt es zu einer Phasenseparation. Polyorganosiloxan lagert sich im wesentlichen an partikuläre Bestandteile im Wasser an oder bildet, bedingt durch ein spez. Gewicht < 1,0 g/cm³ einen Siloxanfilm an der Oberfläche. Polyorganosiloxan wird in Kläranlagen, auch wenn eine aerobe biologische Stufe vorhanden ist, daher weder zersetzt noch abgebaut sondern endet fast quantitativ in der festen Phase des Klärschlamms. Studien solcher Schlämme haben gezeigt, dass die hochmolekularen Siloxane dort dann in durchschnittlich 20-30 Tagen depolymerisiert werden (Gravier et al. 2003) und dann wie beschrieben in die Atmosphäre gelangen und dort oxidiert werden.

Grasset und Palla (US 6,020,184) haben beschrieben, dass auch in wässrigen Systemen ein Abbau von polymerem Siloxan stattfinden kann. Dazu wird eine wässrige Polyorganosiloxan - Suspension mit einem biologisch verwertbaren Co-Substrat wie Glucose versetzt und mit einem Pilz der Gattung Phanaerochaete oder Aspergillus beimpft und aerob inkubiert. Unter diesen Bedingungen findet auch in wässrigen Systemen in 60 Tagen ein Abbau von bis zu 80 % des polymeren PDMS statt. Es ist bekannt, dass die verwendeten Pilze Glukose zunächst nicht vollständig oxidieren sondern organische Säuren produzieren. Bei den entsprechenden pH-Werten von 2,5 - 4,5 findet eine saure Hydrolyse des PDMS zu niedermolekularen Bestandteilen statt. Ein direkter biologischer Abbau des PDMS wird nicht beschrieben.

Flüchtige niedermolekulare Abbauprodukte von PDMS werden hauptsächlich in der Atmosphäre endoxidiert, ein kombinierter biologisch-, chemischer Abbau unter aeroben Bedingungen ist zwar beschrieben (Graiver et al. 2003), ist aber in der Praxis nicht von Bedeutung, da die Verdampfungsrate von flüchtigen Organosiliconen 2 - 20 mal größer ist als die biologische Abbaurate. Eine Akkumulation von niedermolekularen Organosiliconen in oberflächennahen und gut durchlüfteten Böden und Sedimenten findet daher nicht statt, allerdings kann es in tieferen Sedimentschichten und nicht durchlüfteten Böden dennoch zu einer Akkumulation solcher Verbindungen kommen.

Grümping, R. et al., "Microbial Degradation of Octamethylcyclotetrasiloxane", Applied and Environmental Microbiology, Bd. 65, Nr. 5, Mai 1999, Seiten 2276-2278 beschreiben ein Verfahren zum anaeroben biologischen Abbau von Octamethylcyclotetrasiloxan, wobei Dimethylsilandiol entsteht, welches dann ebenfalls abgebaut wird. Der Abbau des Octamethyltetrasiloxans erfolgt dabei sehr langsam.

Aufgabe der vorliegenden Erfindung ist es, ein Verfahren bereitzustellen, mit dem eine Masse enthaltend Silicium Kohlenstoff Einfachbindungen, vorzugsweise Polyorganosiloxane, wie z.B. PDMS oder organofunktionelle Siloxane, oder Organosilane insbesondere Organosilanole oder über chemische Depolymerisation daraus gebildete Bruchstücke, biologisch schneller abgebaut werden kann als aus dem Stand der Technik bekannt.

Die Aufgabe wird gelöst durch ein Verfahren, welches dadurch gekennzeichnet ist, dass eine Mischung aus einer Masse enthaltend Silicium Kohlenstoff Einfachbindungen und eine Mikroorganismenpopulation unter anaeroben oder mikroaeroben Bedingungen unter Zusatz eines alternativen Elektronenakzeptors inkubiert wird, wobei der alternative Elektronenakzeptor Nitrat ist.

Bei der Masse enthaltend Silicium Kohlenstoff Einfachbindungen handelt es sich vorzugsweise um eine Masse enthaltend Polyorganosiloxane, organofunktionelle Siloxane, Organosilane oder aus diesen Verbindungen gebildete Bruchstücke. Vorzugsweise handelt es sich bei der Masse um eine Flüssigkeit oder einen Feststoff.

Bei den Verbindungen, die mit dem erfindungsgemäßen Verfahren bevorzugt abgebaut werden, handelt es sich vorzugsweise um Verbindungen der Formeln (1 bis 3)
(1) HO(SiR₂O)ₚH mit p≥1,
(2) R₃SiO(SiR₂O)_{q}SiR₃ mit q≥0,
(3) (SiR₂O)ᵣ mit r=3-10, oder
   ein Mischpolymerisat aus Einheiten der Formeln HOR₂SiO_{½}, R₃SiO_{½}, R₂SiO, RSi(OH)O, RSiO_{3/2} und HOSiO_{3/2}, oder
   ein Organosiloxanharz aus Einheiten der Formel [R₃SiO_{1/2]} und [SiO_{4/2}], welche noch zusätzliche Si-gebundene OH-Gruppen enthalten,
   wobei R, R₂ und R₃ jeweils gleich oder verschieden sein können und einen einwertigen, linearen oder cyclischen, verzweigten oder unverzweigten gegebenenfalls substituierten Kohlenwasserstoffrest bedeuten.

Der alternative Elektronenakzeptor dient dazu, die von der Mikroorganismenpopulation bei der Oxidation einer Si-R Bindung (wobei R ein einwertiger organischer Rest, vorzugsweise ein einwertiger Alkyl oder Arylrest ist) aufgenommenen Elektronen zu übernehmen und der Mikroorganismenpopulation damit im Rahmen einer anaeroben Atmung die Gewinnung von Energie aus der Substratoxidation zu ermöglichen.

Der alternative Elektronenakzeptor liegt in der Mischung bevorzugt in einer Konzentration von 0,1 - 100 mM vor. Besonders bevorzugt wird der Elektronenakzeptor derart zugesetzt, dass er in einer Konzentration zwischen 1 - 100 mM vorliegt.

Unter mikroaeroben Bedingungen sind Bedingungen zu verstehen, bei denen weniger als 5 % freier oder gelöster Sauerstoff in der Mischung vorhanden ist. Bevorzugt handelt es sich um Bedingungen, bei denen weniger als 1 % freier oder gelöster Sauerstoff in der Mischung vorhanden ist. Besonders bevorzugt sind Bedingungen, bei denen weniger als 250 ppm freier oder gelöster Sauerstoff in der Mischung vorhanden ist.

Mikroaerobe oder anaerobe Bedingungen können zum Beispiel durch technische Verfahren wie Gasaustausch oder chemischen Verbrauch von Restsauerstoff erreicht werden. Bevorzugt werden mikroaerobe oder anaerobe Bedingungen hergestellt, indem vorhandener Sauerstoff durch die vorhandene Mikroorganismenpopulation verbraucht wird und die Zufuhr von weiterem Sauerstoff unterdrückt wird. Besonders bevorzugt werden die mikroaeroben oder anaeroben Bedingungen erreicht, indem das erfindungsgemäße Verfahren in einem geschlossenem Gefäß wie zum Beispiel einem Faulturm in einer Kläranlage durchgeführt wird.

Bei der Mikroorganismenpopulation handelt es sich vorzugsweise um eine Population, wie sie im Klärschlamm oder in einer Kläranlagen oder in einem Bodensediment vorhanden ist. Vorzugsweise handelt es sich um eine Mikroorganismenpopulation, die unter anaeroben Bedingungen wächst, besonders bevorzugt unter diesen Bedingungen ein optimales Wachstum zeigt.

In erfindungsgemäßen Verfahren können Mikroorganismenpopulationen extern zugesetzt werden, oder es können bereits in der Mischung (Klärschlamm, Boden etc.) vorhandene Mikroorganismen verwendet werden.

Das erfindungsgemäße Verfahren benötigt im Gegensatz zu dem in US 6,020,184 offenbarten Verfahren keine weiteren oxidierbaren Substrate (Co-Substrate) wie beispielsweise Kohlenhydrate, z.B. Glukose.

Bevorzugt sind Verfahren, bei denen keine oxidierbaren Cosubstrate zugesetzt werden. Besonders bevorzugt sind solche Verfahren, bei denen keine Cosubstrate im Ansatz vorhanden sind und der Ansatz daher aus den genannten Komponenten besteht.

Das Verfahren wird vorzugsweise bei einer Temperatur von 20 °C bis 80 °C, bevorzugt bei einer Temperatur von 30 °C bis 70 °C, insbesondere bevorzugt bei einer Temperatur von 40 °C bis 60 °C durchgeführt.

Die Inkubation erfolgt vorzugsweise über einen Zeitraum von 1 bis 200 h, bevorzugt 10 bis 150 h, insbesondere bevorzugt 24 bis 100 h.

Das erfindungsgemäße Verfahren ist geeignet, Polyorganosiloxane wie z.B. PDMS oder organofunktionellen Siloxane, und Organosilane insbesondere Organosilanole kontinuierlich (d.h. bei permanentem Zustrom von neuem Substrat und gleichzeitigem Austrag von abgebauten Produkten) oder batch-weise (d.h. in einem Ansatz ohne weiteren Zustrom von neuem Substrat) abzubauen.

Im erfindungsgemäßen Verfahren können die Polyorganosiloxane oder Organosilane vor dem anaeroben Abbau bereits hydrolytisch, z.B. durch Behandlung mit Säure oder Base, vorhydrolysiert worden sein.

Das erfindungsgemäße Verfahren funktioniert beispielsweise in einer Kläranlage, in Sedimenten oder in anderen aquatischen oder terrestrischen Kompartimenten. So kann das erfindungsgemäße Verfahren zum Beispiel in einer anaeroben Stufe in einer Abwasserbehandlungsanlage eingesetzt werden oder es kann eingesetzt werden, um in terrestrischen oder aquatischen, sauerstoffarmen oder sauerstofffreien Kompartimenten vorhandenes Polyorganosiloxan, oder Organosilan oder über chemische Depolymerisation daraus gebildete Bruchstücke abzubauen.

Das folgende Beispiel dient der weiteren Erläuterung der Erfindung.

### Beispiel 1 Abbau von Dimethylsilandiol (DMSD )

Klärschlamm aus einer kommunalen Kläranlage wurde aus dem laufenden Betrieb unter sauerstofffreien Bedingungen (N₂ haltige Probengefäße) entnommen. Zur Abtrennung störender Substrate wurde die Zellmasse in dem 5-fachen Volumen eines sauerstofffreien Puffers (50 mmol/l Kaliumphosphat pH 6,8) resuspendiert und abzentrifugiert. Sauerstofffreie Lösungen wurden durch Entgasen der Lösung und Spülen mit gasförmigem Stickstoff hergestellt.

Der Vorgang (Resuspendieren / Abzentrifugieren) wurde 3-mal wiederholt.

Die gewaschene sauerstofffreie Zellmasse wurde unter Ausschluß von Sauerstoff in Schüttelkolben mit Kulturmedium überführt (10 g feuchte Zellmasse auf 100 ml Medium). Für Kontrollansätze wurde der Klärschlamm autoklaviert und damit inaktiviert. Die Kultivierung erfolgte in einem Minimalmedium (SM1) ohne den Zusatz komplexer Nährstoffe. Das Kulturmedium setzte sich wie folgt zusammen:

| Salz: | [g/l] |
|---|---|
| CaCl₂ x 2 H₂O | 0,0147 |
| MgSO₄ x 7 H₂O | 0,3 |
| KH₂PO₄ | 3,0 |
| K₂HPO₄ | 12,0 |
| (NH₄)₂SO₄ | 5,0 |
| NaCl | 0,1 |
| FeSO₄ x 7 H₂O | 0,002 |
| Na₃-Citrat x 2 H₂0 | 1,0 |

| Spurenelemente: | [mg/l] |
|---|---|
| Na₂MO₄ x 2 H₂O | 0,15 |
| CoCl₂ x 6 H₂O | 0,7 |
| CuSO₄ x 5 H₂O | 0,25 |
| MnCl₂ x 4 H₂O | 1,6 |
| ZnSO₄ x 7 H₂O | 0,3 |

Als Elektronenakzeptor wurde darüber hinaus noch 5 g/l KNO₃ zugegeben. Als einzige Kohlenstoffquelle wurde abschließend in das Medium Dimethylsilandiol (wasserlösliche; Molgewicht 92 g/l) zugegeben. Die Konzentration in den Ansätzen betrug 1 mmol/l. Die Kultivierung erfolgte unter anaeroben Bedingungen auf Rundschüttlern bei einer Temperatur von 30°C. Die Gesamtkultivierungszeit betrug 11 Tage. Proben des Kulturmemediums wurden in regelmäßigen Abständen entnommen und sofort analysiert. Die Probenentnahme erfolgte dabei ebenfalls unter anaeroben Bedingungen (Glove Box, N2 Atmosphäre) direkt aus den Schüttelkolben.

Analytik: In Versuchen mit DMSD als Substrat wurde die Änderung der Konzentration von DMSD in der wässrigen Phase mittels Protonen Kernresonanzspektroskopie (1H-NMR) bestimmt. Gut geeignet ist dabei das intensive Signal bei 0,164 ppm. Proben (0,9 ml) aus den Kulturansätzen wurden dazu direkt (durch den Stopfen) aus dem Gefäß entnommen, mit Standard versetzt (TSP in D20; TSP = 3-(Trimethylsilyl)-propionsäure-D4 Natriumsalz.) und im Spektrometer analysiert. Über das bekannte Standardsignal kann das DMSD Signal genau quantifiziert werden.

**Ergebnis der Ansätze mit DMSD (mg/l)**

| | | | | | |
|---|---|---|---|---|---|
| Inkubationszeit (Tage) | 0 | 2 | 4 | 7 | 11 |
| Ansatz Klärschlamm DMSD (mg/l) | 90 | 85 | 73 | 64 | 55 |
| Kontrollansatz inaktivierter Klärschlamm DMSD (mg/l) | 87 | 87 | 86 | 83 | 79 |

Gegenüber dem Kontrollansatz (- 9% in 11 Tagen) zeigt sich eine deutliche Abnahme der DMSD Menge im anaerob inkubierten Ansatz mit Klärschlamm (- 39% in 11 Tagen).

### Beispiel 2 Abbau von Octamethylcyclotetrasiloxan (D4)

Der Versuch wurde entsprechend Beispiel 1 durchgeführt.

Es wurden jeweils fünf Kolben mit Klärschlamm und fünf Kolben mit inaktiviertem Klärschlamm angesetzt. Als Kohlenstoffquelle wurde dem Medium Octamethylcyclotetrasiloxan (D4) zugegeben. Das Siloxan ist mit Wasser nicht mischbar und bildet zunächst einen öligen Film auf der Kulturoberfläche. Mit fortschreitender Kultivierungsdauer wird das Siloxanöl in der Kultur emulgiert. Es bilden sich größere Zellaggregate.

Die Kultivierung erfolgte entsprechend Beispiel 1 in dem dort angegebenen Mineralmedium (SM1) mit 5 g/l KNO₃ als Elektronenakzeptor. Als einzige Kohlenstoffquelle wurde den Ansätzen Octamethylcyclotetrasiloxan (D4) zugegeben (1 ml auf 100 ml Medium). Nach unterschiedlich langer Inkubation wurde der D4 - Gehalt der Ansätze bestimmt.

### Analytik von D4

Der gesamte Ansatz wurde 3 mal mit 50 ml Pentan extrahiert, zur Erleichterung der Phasentrennung wurde jeweils zentrifugiert. Die Pentanphasen wurden vereinigt und D4 direkt mittels Gaschromatographie (Gerät hp5890_1i; Hewlett Packard) quantitativ bestimmt. Die gaschromatographische Bestimmung erfolgte mit einer 30 m Kapillare (Hewlett Packard HP-1 Nr. 59026323) mit Stickstoff als Trägergas. Temperaturprogramm: 50°C(5 min) - 270°C mit 20°C/min. Detektion mittels FID bei 300°C. Die Quantifizierung der erhaltenen Signalpeaks wurde mit entsprechenden Standardlösungen durchgeführt.

**Ergebnis der Ansätze mit D4 (ppm)**

| | | | | | |
|---|---|---|---|---|---|
| Inkubationszeit (Tage) | 0 | 2 | 4 | 7 | 11 |
| Ansatz Klärschlamm D4 (ppm) | 7805 | 7050 | 6900 | 6732 | 6532 |
| Kontrollansatz inaktivierter Klärschlamm D4 (ppm) | 8056 | 8010 | 7944 | 7770 | 7557 |

Gegenüber dem Kontrollansatz (- 6,2 % in 11 Tagen) zeigte sich eine deutliche Abnahme der D4 Menge im anaerob inkubierten Ansatz mit Klärschlamm (- 16,3 % in 11 Tagen).

## Patentansprüche

1. Verfahren zum biologischen Abbau einer Masse enthaltend eine Silicium Kohlenstoff Einfachbindung **dadurch gekennzeichnet, dass** eine Mischung aus der Masse und einer Mikroorganismenpopulation unter anaeroben oder mikroaeroben Bedingungen unter Zusatz eines alternativen Elektronenakzeptors inkubiert wird, wobei der alternative Elektronenakzeptor Nitrat ist.

2. Verfahren gemäß Anspruch 1 **dadurch gekennzeichnet, dass** die Masse enthaltend Silicium Kohlenstoff Einfachbindung eine Masse enthaltend Polyorganosiloxane, organofunktionelle Siloxane, Organosilanole oder deren Bruchstücken ist.

3. Verfahren gemäß einem der Ansprüche 1 oder 2 **dadurch gekennzeichnet, dass** die anaeroben oder mikroaeroben Bedingungen so gewählt sind, dass weniger als 5 % freier oder gelöster Sauerstoff im Ansatz vorhanden ist.

4. Verfahren gemäß Anspruch 3, **dadurch gekennzeichnet, dass** weniger als 1 % bevorzugt weniger als 250 ppm freier oder gelöster Sauerstoff im Ansatz vorhanden ist.

5. Verfahren gemäß einem der Ansprüche 1 bis 4, **dadurch gekennzeichnet, dass** der alternative Elektronenakzeptor in einer Konzentration von 0,1 - 100 mM eingesetzt wird.

6. Verfahren gemäß einem der Ansprüche 1 bis 5, **dadurch gekennzeichnet, dass** es bei einer Temperatur von 20 bis 80 °C, bevorzugt bei einer Temperatur von 30 bis 70 °C, insbesondere bevorzugt bei einer Temperatur von 40 bis 60 °C durchgeführt wird.

7. Verfahren gemäß einem der Ansprüche 1 bis 6, **dadurch gekennzeichnet, dass** die Inkubation über einen Zeitraum von 1 bis 200 h, bevorzugt 10 bis 150 h, insbesondere bevorzugt 24 bis 100 h erfolgt.

## Claims

1. Method for the biological decomposition of a material comprising a silicon-carbon single bond, **characterized in that** a mixture of the material and a microorganism population is incubated under anaerobic or microaerobic conditions with addition of an alternative electron acceptor, the alternative electron acceptor being nitrate.

2. Method according to Claim 1, **characterized in that** the material comprising a silicon-carbon single bond is a material comprising polyorganosiloxanes, organofunctional siloxanes, organosilanols or their fragments.

3. Method according to Claim 1 or 2, **characterized in that** the anaerobic or microaerobic conditions are selected such that less than 5% of free or dissolved oxygen is present in the batch.

4. Method according to Claim 3, **characterized in that** less than 1%, preferably less than 250 ppm, of free or dissolved oxygen is present in the batch.

5. Method according to one of Claims 1 to 4, **characterized in that** the alternative electron acceptor is used in a concentration of 0.1-100 mM.

6. Method according to one of Claims 1 to 5, **characterized in that** it is carried out at a temperature of 20 to 80°C, preferably at a temperature of 30 to 70°C, in particular preferably at a temperature of 40 to 60°C.

7. Method according to one of Claims 1 to 6, **characterized in that** the incubation proceeds over a period of 1 to 200 h, preferably 10 to 150 h, in particular preferably 24 to 100 h.

## Revendications

1. Procédé pour la dégradation biologique d'une masse contenant une simple liaison silicium-carbone, **caractérisé en ce qu'**on incube un mélange de la masse et d'une population de microorganismes dans des conditions anaérobies ou microaérobies avec addition d'un accepteur d'électrons alternatif, l'accepteur d'électrons alternatif étant un nitrate.

2. Procédé selon la revendication 1, **caractérisé en ce que** la masse contenant une simple liaison silicium-carbone est une masse contenant des polyorganosiloxanes, des siloxanes organofonctionnels, des organosilanols ou leurs fragments.

3. Procédé selon la revendication 1 ou 2, **caractérisé en ce que** les conditions anaérobies ou microaérobies sont choisies de manière telle qu'il existe moins de 5% d'oxygène libre ou dissous dans la charge.

4. Procédé selon la revendication 3, **caractérisé qu'**il existe moins de 1%, de préférence moins de 250 ppm d'oxygène libre ou dissous dans la charge.

5. Procédé selon l'une quelconque des revendications 1 à 4, **caractérisé en ce que** l'accepteur d'électrons alternatif est utilisé en une concentration de 0,1-100 mM.

6. Procédé selon l'une quelconque des revendications 1 à 5, **caractérisé en ce qu'**il est réalisé à une température de 20 à 80°C, de préférence à une température de 30 à 70°C, de manière particulièrement préférée à une température de 40 à 60°C.

7. Procédé selon l'une quelconque des revendications 1 à 6, **caractérisé en ce que** l'incubation est réalisée sur un laps de temps de 1 à 200 h, de préférence de 10 à 150 h, de manière particulièrement préférée de 24 à 100 h.
